# EUROPEAN PATENT SPECIFICATION

(11) **EP 0 638 077 B1**
(45) Date of publication and mention of the grant of the patent: **19.09.2001**
(21) Application number: 93909073.4
(22) Date of filing: 21.04.1993
(51) Int. Cl.: C07D 307/93, C12P 41/00, C12P 17/04, C07C 35/06

(54) **CHIRAL CYCLOPENTENE DERIVATIVES AND THEIR PREPARATION**
CHIRALE CYCLOPENTENDERIVATE UND IHRE HERSTELLUNG
DERIVES DE CYCLOPENTENE CHIRAUX ET LEUR PREPARATION

(30) Priority: 21.04.1992 WO PCT/GB92/00730; 25.09.1992 GB 9220253
(43) Date of publication of application: 15.02.1995
(73) Proprietor: Chirotech Technology Limited, Cambridge CB4 4WE (GB)
(72) Inventor: ROBERTS, Stanley Michael, Kenton, Devon EX6 8NH (GB); OLIVO, Horacio F., Exeter, Devon EX1 2PU (GB); McCAGUE, Raymond, Milton, Cambridgeshire CB4 6EE (GB)
(74) Representative: Perry, Robert Edward
(86) International application number: GB9300826
(87) International publication number: WO9321175

(56) References cited:
- EP-A- 0 212 956
- EP-A- 0 267 878
- EP-A- 0 271 433
- EP-A- 0 501 310
- WO-A-92/18444
- CHEMISCHE BERICHTE vol. 109, 1976, WEINHEIM pages 444 - 454 REINER SUSTMANN ET. AL.
- JOURNAL OF THE CHEMICAL SOCIETY. PERKIN TRANSACTIONS 1 no. 10, October 1991, CAMBRIDGE pages 2603 - 2604 ELIZABETH A. SAVILLE-JONES ET. AL. cited in the application
- TETRAHEDRON LETTERS vol. 32, no. 51, 16 December 1991, OXFORD pages 7529 - 7530 ANDR LUBINEAU ET. AL. cited in the application
- JOURNAL OF THE CHEMICAL SOCIETY. CHEMICAL COMMUNICATIONS. no. 4, 15 February 1992, CAMBRIDGE pages 368 - 370 PAUL A. GRIECO cited in the application

## Description

### Field of the Invention

This invention relates to chiral cyclopentene derivatives and also to their preparation and use. In single enantiomer form, hydroxylated cyclopentene derivatives are valuable precursors to carbocyclic nucleosides and to cholesterol-lowering agents.

### Background of the Invention

WO-A-9218444 describes such enantiomers, of 2-hydroxymethyl-4-hydroxycyclopentene, and their synthesis. The first synthetic step is a Prins addition reaction of formaldehyde and formic acid onto cyclopentadiene. The adduct is a mixture of isomers which are separated chromatographically as appropriately protected derivatives and resolved in enzyme-mediated transformations.

The same reaction is described by Sackville-Stones et al, JCS Perkin Trans. I (1991) 2603. Enantiomers of 3-hydroxymethyl-2-hydroxycyclopentene are described as a minor product. The chiral cyclopentene derivatives are useful as synthons in the preparation of carbocyclic nucleosides such as Carbovir. However, a difficulty with this methodology is the problematic requirement for separation of regioisomers and diastereoisomers in addition to the enantiomers.

Lubineau et al, Tet. Lett. 32(51):7529-30 (1991), describe the hetero-Diels-Alder reaction of cyclopentadiene with glyoxylic acid, to give an α-hydroxy-γlactone, specifically 4-hydroxy-2-oxabicyclo[3.3.0]oct-7-en-3-one. This adduct has been used, as a mixture of diastereoisomers, in the synthesis of (±)-sesbanimides; see Grieco et al, JCS Chem. Commun. (1992) 568.

### Summary of the Invention

The present invention is based on the discovery that the known lactone (which is produced from starting materials that are cheap and readily-available) can be resolved by biotransformation and that the resultant enantiomer can be used to produce the known hydroxylated cyclopentene derivatives in enantiomeric form, i.e. ready for use as a synthon in the preparation of a desired enantiomer of Carbovir or other carbocyclic nucleosides.

### Description of the Invention

As indicated above, the adduct of cyclopentadiene and glyoxylic acid has previously been produced as a racemate. The present invention provides methods for its transformation into single or at least substantially pure (e.g. more than 50% ee) enantiomer material of greater utility.

In one aspect of the invention, the adduct (which is a mixture of diastereoisomers owing to the configuration of the secondary hydroxyl fraction) is resolved to give a desired enantiomer. The adduct may be recrystallised to provide a single diastereoisomer that is the major component of the original mixture, but this is often unnecessary.

One resolution procedure comprises treatment with an acyl donor (e.g. vinyl acetate) and a suitable biocatalyst in an appropriate solvent (e.g. heptane). The biocatalyst may be a lipase such as Pseudomonas fluorescens (pfl), P. lipoprotein or Candida cylindracea lipase.

Alternatively, resolution may be conducted by acylation (e.g. with butyric acid) then hydrolysis with a suitable biocatalyst in an aqueous system. The butyrate is transformed at substantially the same rate as the acetate, although the aqueous biotransformation of the butyrate is faster than the esterification with vinyl acetate. In general, it is preferred that the acylate has up to 6 C atoms.

The transformation leaves one enantiomer as the secondary alcohol and the other as its acyl derivative. They may be separated by conventional methods. If the biocatalyst preferentially transforms the endo rather than exo adduct, as is the case for pfl, the endo diastereoisomer being the major component and the fact that endo-hydroxy-lactone can be recrystallised from exo in the optically-active product means that it is unnecessary to recover pure endo-racemate.

As the second feature of the invention, resolution of the adduct as the single diastereoisomer or a mixture of the two, is by means of a biocatalyst in aqueous medium that effects hydrolysis of the lactone function in a manner specific for one enantiomer. That leaves unconsumed lactone and ring-opened carboxylate that can be separated through solvent extraction.

Conversions of the resolved adduct into a synthetically useful diol, e.g. as described by Sackville-Stones et al, supra, may be accomplished by a sequence of standard chemical transformations. The reaction conditions for such transformations are illustrated in the Examples, below, with reference to the accompanying Scheme. Exemplifying reagents and conditions for that Scheme are: (i) pfl, vinyl acetate; (ii) LiAlH₄, THF; (iii) NaIO₄, Et₂O-H₂O; (iv) NaBH₄, MeOH; (v) Ph₃CCl, Et₃N, Me₂NC₅H₄,CH₂Cl₂; (vi) Ac₂O, pyridine; (vii) 2-amino-6-chloropurine, NaH, DMF, (Ph₃P)₄Pd, THF. The three operations of steps ii, iii and iv be conducted without purification of the intermediates. As an alternative to the given sequence of steps ii, iii and iv, the hydroxy-lactone may be reacted first with a carbanionic nucleophile such as RLi, to give a compound of formula IV (see claim 6; IV is a generalised version of **4**). Effective dehydroxymethylation, to give **5**, may be conducted using KIO₄ and NaBH₄.

Tritylation of the primary hydroxy group in the diol (-)-**5** gives (-)-**6** and acetylation of the secondary hydroxy group gives the ester (-)-**7** [α]²⁵_{D} -85 (c 1.0, CHCl₃). Compounds of type **7** are excellent precursors of 2',3'-dideoxydidehydrocarbocyclic nucleosides through the use of Trost-style organopalladium chemistry. Thus, reaction of the allylic acetate (-)-**7** with 2-amino-6-chloropurine and sodium hydride in the presence of tetrakis(triphenylphosphine)palladium(0) gives the cyclopentene derivative (-)-**8** [α]²⁵_{D} -75 (c 1.0, CHCl₃), a known precursor of the anti-HIV agent Carbovir (-)-**9**. Interest in this anti-viral substance remains at a high level. The appropriate preparation of Carbovir is described by Evans et al, JCS Perkin I (1992) 589; see also Sackville-Stones et al, supra.

### Example 1

Cyclopentadiene (240 cm³, 2.87 mol), aq. glyoxylic acid (225 cm³, 50% w/v solution, 2.01 mol) and water (800 cm³) were vigorously stirred at 0°C to room temperature for 4 days. The solution was extracted with heptane (4 x 250 cm³), to remove unchanged cyclopentadiene, saturated with sodium chloride and further extracted with ethyl acetate (12 x 500 cm³). The combined organic layers were concentrated to 1.5 dm³, cooled to 0°C and washed with cold saturated aqueous sodium hydrogen carbonate (2 x 200 cm³), to remove unchanged glyoxylic acid. The aqueous solution was extracted with ethyl acetate (3 x 500 cm³). The combined organic layers were dried (Na₂SO₄), filtered and evaporated to give the lactones (±)-**1** and (±)-**2** as a yellow mobile oil which crystallised on storage (ca. 65% yield). NMR spectroscopy showed the ratio **1:2** was 4:1. The crude product was triturated with methyl tert-butyl ether to remove the oily lactone (±)-**2**, and the lactone (±)-**1** (88 g, 628 mmol) was isolated as a sand-coloured crystalline solid (greater than 95% pure by NMR spectroscopy) which could be used in the next step. Recrystallisation from diethyl ether gave pure (±)-4-hydroxy-2-oxabicyclo[3.3.0]oct-7-en-3-one **1**.

Alternatively, chromatography of the crude product over silica using hexane in ethyl acetate (ratio 2:3) as eluant to give, in the first fractions, the lactone (±)-**2**; νₘₐₓ/cm⁻¹ 3427, 1764, 1615, 1184, 1116 and 986; δ_{H}(CDCl₃) 6.1 (1 H, m, 7-H or 8-H), 5.9 (1 H, m, 8-H or 7-H), 5.55 (1 H, m, 1-H), 4.15 (1 H, d, J 7, 4-H). 3.5 (1 H, br, s, OH) and 3.05-2.55 (3 H, m, 5-H and 2 x 6-H) ; δ_{c}(CDCl₃) 178.2, 136.9, 129.2, 87.5, 74.3, 44.3 and 36.6. Later fractions contained the lactone (±)-1 m.p. 68-69°C; νₘₐₓ/cm⁻¹ 3443, 3023, 1765, 1615 and 1134; δ_{H}(CDCl₃) 6.33 (1 H, m, 7-H or 8-H), 5.93 (1 H, m, 8-H or 7-H), 5.33 (1 H, dt, J 6,2, 1-H), 4.72 (1 H, d, J 9, 4-H), 3.5 (1 H, br, s, OH) and 3.21-2.45 (3 H, m, 5-H and 2 x 6-H); δ_{c}(CDCl₃) 177.6, 140.9, 127.4, 86.4, 67.0, 40.4 and 30.7 (Found: C, 59.9; H, 5.7. C₇H₈O₃ requires C, 60.0; H, 5.75%).

The hydroxy-lactones **1** and **2** are thus produced in the ratio 4:1 on a large scale (10-250 g) (crude yield ca. 65%). The compounds were separated by chromatography over silica and the disposition of 4-OH in the epimers was elucidated by NMR spectroscopy. The epimer **1** crystallises from the crude product. The minor component **2** can then be removed by trituration whereupon **1** can be obtained in greater than 95% purity in 31% yield. Recrystallisation from diethyl ether gave pure **1**. Confirmation of the proposed structure for the lactone **1** was obtained by X-ray crystallography.

### Example 2

This Example illustrates hydrolysis of the butyrate of the hydroxy-lactone-(±)-4-hydroxy-2-oxabicyclo[3.3.0]oct-7-en-3-one.

The (+)-hydroxy-lactone ester (2.391 kg, 4.5:1 ratio of endo:exo isomers) was stirred in phosphate buffer (20 L, 0.1 M KH₂PO₄) at 25°C, and the pH adjusted to 6.6 using 10 M NaOH. Lipase (Amano PS, 120 g) was added as a slurry in IL phosphate buffer, then the mixture stirred well to disperse the ester. The mixture was controlled at pH 6.6 by automatic addition of 10 M NaOH. After addition of 4.85 mol hydroxide, 10% w/v NaCl was added, then excess butyrate ester and undissolved solids were removed by centrifugation. The supernatant was extracted 3 times with a equal volume of ethyl acetate, the organic extracts dried (MgO₄), then concentrated under reduced pressure to 5% of the original volume. Methyl tert-butyl ether was added to induce crystallisation and after chilling to 5°C, the crystals were harvested by filtration, then recrystallised from dichloromethane, yielding 396 g (-)-hydroxy-lactone of >99% ee. The ee was determined as the p-toluate ester, separating enantiomers on a 25 cm Pirkle L-leucine column (4% ethanol in heptane as mobile phase, 254 nm, 2 ml/min).

### Example 3

This Example illustrates esterification of (±)-hydroxy-lactone in vinyl acetate.

Vinyl acetate (2 L), (±)-hydroxy-lactone (100 g), and lipase (28 g, Amano PS) were stirred gently at 40°C for 72 hours. The enzyme was recovered by filtration, then the vinyl acetate evaporated under reduced pressure. Methyl tert-butyl ether (250 ml) was added to cause crystallisation of (+)-hydroxy-lactone. This was recovered by filtration, yielding 19 g (+)-hydroxy-lactone of >99% ee. The remaining lactone was partitioned away from the ester in 1:1 toluene:brine (600+600 ml), washing the organic layer twice with brine. The toluene layer was then dried (MgSO₄), and concentrated under reduced pressure, then MeOH (400 ml) and sodium methoxide (2 g) were added. After stirring for 1 h, acetic acid (2 ml) was added, and the methanol evaporated, replacing with ethyl acetate (400 ml). The resulting suspension was filtered through silica gel, then concentrated, and crystallisation induced by addition of methyl tert-butyl ether. The crystals were recovered by filtration, and dried, yielding (-)-hydroxy-lactone (18 g) of 98% ee.

The hydroxy-lactone **1** was thus resolved by an enzymecatalysed process. In particular, the lactone (±)-**1** was partially acetylated using Pseudomonas fluorescens lipase (pfl) in vinyl acetate.

### Example 4

The (-)-hydroxy-lactone derived from the biocatalytic hydrolysis was converted into the diol (-)-**5** in three steps. Thus, lithium aluminium hydride (1.4 g) was suspended in tetrahydrofuran (50 ml) and the mixture heated to reflux. The (-)-hydroxy-lactone (5.32 g) in tetrahydrofuran (50 ml) was then added over 20 min. The mixture was allowed to cool to ambient and quenched by the addition of saturated aqueous sodium sulphate solution (15 ml). The solid was removed by filtration and washed with tetrahydrofuran (5 x 10 ml). The combined filtrate and washings were concentrated under reduced pressure and the residual crude triol **4** taken up in 1:1 methyl tert-butyl ether: saturated brine (100 ml). This biphasic mixture was cooled to 0°C and sodium periodate (8.52 g) added in portions over 10 min. The resulting mixture was stirred for 1 h, then the solids removed by filtration and washed with methyl tert-butyl ether (50 ml). The filtrates were concentrated under reduced pressure and the residue dissolved in ethanol (50 ml). The solution was cooled to 0°C and sodium borohydride (1.41 g) added in small portions over 2 min. The resulting mixture was stirred at 0°C overnight then sodium chloride added to saturate the aqueous layer which was extracted with ethyl acetate (3 x 100 ml). The organic layers were combined, dried over sodium sulphate and concentrated under reduced pressure to leave crude diol (-)-**5** (3.27 g). Distillation at 1 mm Hg is used to purify the diol and generally gives a yield of about 35% from the (-)-hydroxy-lactone.

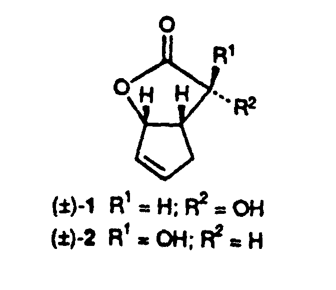

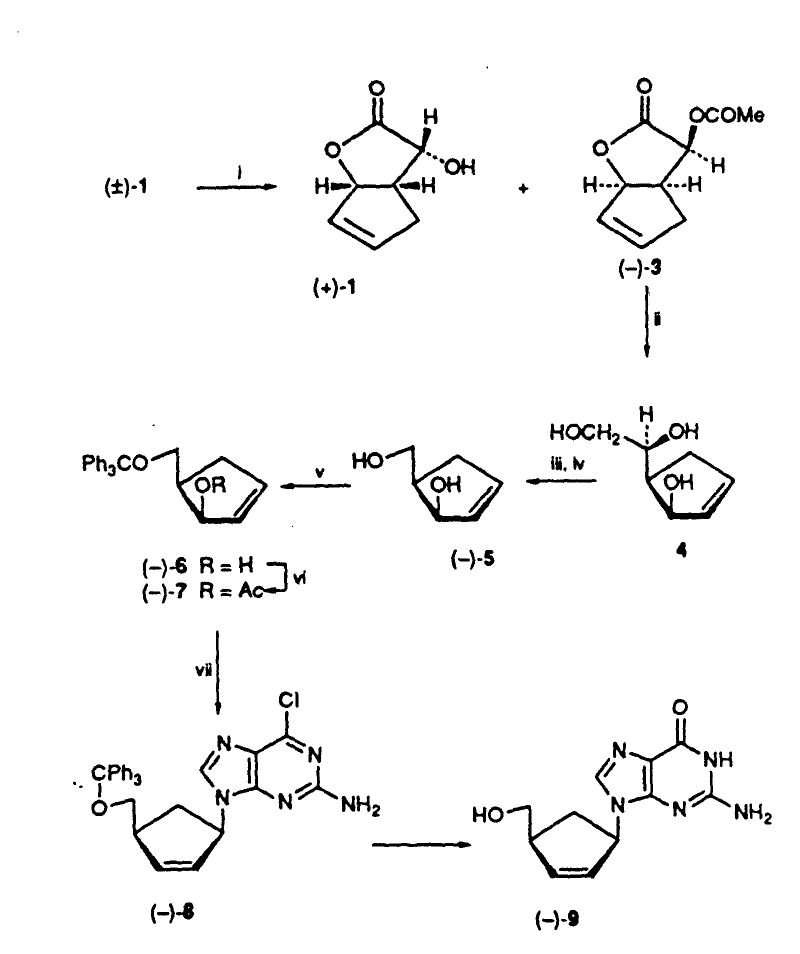

## Claims

1. An enantiomer of the lactone 4-hydroxy-2-oxabicyclo-[3.3.0]oct-7-en-3-one or an acylate thereof, substantially free of the other enantiomer.

2. An enantiomer according to claim 1, which is the (-)-enantiomer.

3. A process for the resolution of a mixture of enantiomers of the lactone or acylate defined in claim 1 or claim 2, which comprises biocatalytic enantiospecific hydrolysis of the acylate or biocatalyst-mediated enantiospecific acylation of the non-acylated lactone.

4. A process for the resolution of a mixture of enantiomers of the lactone or acylate defined in claim 1 or claim 2, which comprises biocatalytic enantiospecific hydrolysis of the lactone function.

5. A process according to claim 3 or claim 4, wherein the biocatalyst is a lipase.

6. A process for preparing an enantiomer of a compound of the formula IV 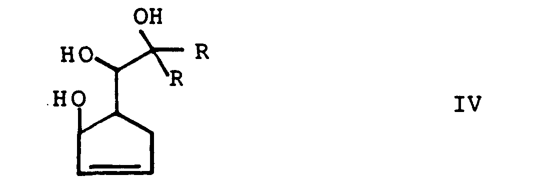 wherein each R is H or alkyl, which comprises subjecting an enantiomer of claim 1 or claim 2 to treatment with a carbanionic nucleophile capable of introducing the group R.

7. A process according to claim 6, wherein the compound of formula IV is 3-(1,2-dihydroxyethyl)-2-hydroxycyclopentene.

8. A process according to claim 6 or claim 7, wherein the nucleophile is RLi.

9. A process for preparing 3-(1,2-dihydroxyethyl)-2-hydroxycyclopentene in enantiomeric form, which comprises reducing an enantiomer according to claim 1 or claim 2.

10. A process for preparing 3-hydroxymethyl-2-hydroxycyclopentene in enantiomeric form, which comprises a process according to claim 9 and the dehydroxymethylation of the 3-(1,2-dihydroxyethyl)-2-hydroxycyclopentene.

## Patentansprüche

1. Enantiomer des Lactons 4-Hydroxy-2-oxabicyclo[3.3.0]oct-7-en-3-on oder eines Acylats davon, im wesentlichen frei von dem anderen Enantiomer.

2. Enantiomer nach Anspruch 1, welches das (-)-Enantiomer ist.

3. Verfahren zur Auftrennung einer Mischung von Enantiomeren des Lactons oder Acylats nach Anspruch 1 oder Anspruch 2, welches die biokatalytische enantiospezifische Hydrolyse des Acylats oder Biokatalysator-vermittelte enantiospezifische Acylierung des nicht-acylierten Lactons umfaßt.

4. Verfahren zur Auftrennung einer Mischung von Enantiomeren des Lactons oder Acylats nach Anspruch 1 oder 2, welches die biokatalytische enantiospezifische Hydrolyse der Lactonfunktion umfaßt.

5. Verfahren nach Anspruch 3 oder 4, worin der Biokatalysator eine Lipase ist.

6. Verfahren zur Herstellung eines Enantiomers einer Verbindung der Formel IV 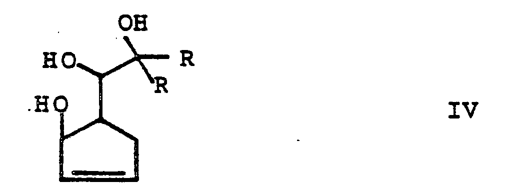 worin jedes R H oder Alkyl ist, welches umfaßt, daß ein Enantiomer nach Anspruch 1 oder Anspruch 2 einer Behandlung mit einem carbanionischen Nukleophil, welches zur Einführung der Gruppe R in der Lage ist, unterworfen wird.

7. Verfahren nach Anspruch 6, worin die Verbindung der Formel IV 3-(1,2-Dihydroxyethyl)-2-hydroxycyclopenten ist.

8. Verfahren nach Anspruch 6 oder Anspruch 7, worin das Nukleophil RLi ist.

9. Verfahren zur Herstellung von 3-(1,2-Dihydroxyethyl)-2-hydroxycyclopenten in enantiomerer Form, welches die Reduktion eines Enantiomers nach Anspruch 1 oder Anspruch 2 umfaßt.

10. Verfahren zur Herstellung von 3-Hydroxymethyl-2-hydroxycyclopenten in enantiomerer Form, welches ein Verfahren nach Anspruch 9 und die Dehydroxymethylierung des 3-(1,2-Dihydroxyethyl)-2-hydroxycyclopentens umfaßt.

## Revendications

1. Enantiomère de la lactone 4-hydroxy-2-oxabicyclo[3.3.0]oct-7-èn-3-one ou d'un de ses acylates, sensiblement exempt de l'autre énantiomère.

2. Enantiomère selon la revendication 1, qui est l'énantiomère (-).

3. Procédé pour la résolution d'un mélange d'énantiomères de la lactone ou d'un de ses acylates selon la revendication 1 ou la revendication 2, qui comprend l'hydrolyse énantiospécifique biocatalysée de l'acylate ou l'acylation énantiospécifique, grâce à un biocatalyseur, de la lactone non acylée.

4. Procédé pour la résolution d'un mélange d'énantiomères de la lactone ou d'un de ses acylates selon la revendication 1 ou la revendication 2, qui comprend l'hydrolyse énantiospécifique biocatalysée de la fonction lactone.

5. Procédé selon la revendication 3 ou la revendication 4, dans lequel le biocatalyseur est une lipase.

6. Procédé pour la préparation d'un énantiomère d'un composé de formule IV 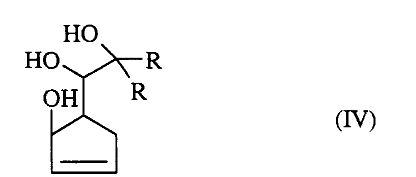 dans lequel chaque R est H ou alkyle, qui comprend la soumission d'un énantiomère selon la revendication 1 ou la revendication 2 à un traitement par un nucléophile carbanionique capable d'introduire le groupe R.

7. Procédé selon la revendication 6, où le composé de formule IV est le 3-(1,2-dihydroxyéthyl)-2-hydroxycyclopentène.

8. Procédé selon la revendication 6 ou la revendication 7, où le nucléophile est RLi.

9. Procédé pour la préparation du 3-(1,2-dihydroxyéthyl)-2-hydroxycyclopentène sous forme énantiomère, qui comprend la réduction d'un énantiomère selon la revendication 1 ou la revendication 2.

10. Procédé pour la préparation du 3-hydroxyméthyl-2-hydroxycyclopentène sous forme énantiomère, qui comprend un procédé selon la revendication 9 et la déshydroxyméthylation du 3-(1,2-dihydroxyéthyl)-2-hydroxycyclopentène.
